# NEUE EUROPÄISCHE PATENTSCHRIFT

(11) **EP 0 302 248 B2**
(45) Veröffentlichungstag und Bekanntmachung des Hinweises auf die Entscheidung über den Einspruch: **10.01.1996**
(45) Hinweis auf die Patenterteilung: 23.10.1991
(21) Anmeldenummer: 88110812.0
(22) Anmeldetag: 06.07.1988
(51) Int. Cl.: A61M 5/28

(54) **Injektionsspritze für medizinische Zwecke**
Injection syringe for medical purposes
Seringue à injection pour usage médical

(30) Priorität: 21.07.1987 DE 3724120; 18.05.1988 DE 3816961
(43) Veröffentlichungstag der Anmeldung: 08.02.1989
(73) Patentinhaber: WASSERBURGER ARZNEIMITTELWERK DR. MADAUS GMBH & CO. KG, D-83512 Wasserburg (DE)
(72) Erfinder: Lix, Helmut, Dr., & Co. KG Herderstr. 2 D-8090 Wasserburg (DE)
(74) Vertreter: Kinzebach, Werner, Dr.

(56) Entgegenhaltungen:
- EP-A- 0 144 551
- CH-A- 575 765
- US-A- 2 549 417
- US-A- 2 591 046
- US-A- 3 737 973
- US-A- 4 266 557
- US-A- 4 613 326
- DIN-Norm 58 359

## Beschreibung

Die Erfindung betrifft eine Injektionsspritze für medizinische Zwecke mit einem mindestens einen verschiebbaren Spritzenkolben aufweisenden Spritzenzylinder, der an seinem einen Ende ein Nadelansatzstück und an seinem dem Nadelansatzstück gegenüberliegenden Ende eine Öffnung zum Befüllen und zum Einführen des bzw. der Spritzenkolben(s) aufweist.

Diese Spritze soll insbesondere die Möglichkeit bieten, das Arzneimittel in der Spritze im Vakuum zu trocknen oder zu lyophilisieren (gefriertrocknen). Beim Gefriertrocknen werden oft beachtliche Mengen Lösungsmittel im Vakuum abgezogen, was voraussetzt, daß der Behälter, in dem sich das so behandelte Gut befindet, über genügend große Öffnungen verfügt, durch die der Lösungsmitteldampf austreten kann.

Spritzen dieses Typs sind z.B. aus den offengelegten Europäischen Patentanmeldungen 0 191 122 und 0 144 483 und aus der DE-OS 33 39 705 bekannt. In diesen Veröffentlichungen wird zur Lösung des oben erwähnten Problems ein Spritzentyp vorgeschlagen, der am nadelseitigen Ende eine große Öffnung hat, welche erst nach Beendigung der Gefriertrocknung durch Einsetzen eines Nadelansatzstückes verschlossen wird.

Nachteilig an diesen Spritzentypen ist, daß das Einschließen des fertigen Lyophilisates in der Spritze durch Einsetzen des Nadelansatzstückes erfolgt` dabei müssen mehrere Teile montiert werden, wozu aufwendige technische Operationen erforderlich sind, die im sterilen Bereich der Produktionsanlage durchgeführt werden müssen, denn die Spritze darf den Sterilbereich erst dann verlassen, wenn das Lyophilisat in der Spritze dicht verschlossen ist.

Der Erfindung liegt daher die Aufgabe zugrunde, eine Spritze der eingangs geschilderten Art so zu gestalten, daß ein Arzneimittel in der Spritze gefriergetrocknet werden kann und die Spritze mit dem enthaltenen Lyophilisat nach Beendigung der Gefriertrocknung noch sofort in der Gefriertrockenanlage und auf einfache Weise versiegelt werden kann, so daß der Zutritt von Feuchtigkeit in das Lyophilisat verhindert wird, wenn die Gefriertrockenanlage geöffnet wird,

Diese Aufgabe wird erfindungsgemäß durch die Merkmale der Ansprüche 1 und 4 gelöst.

Vorzugsweise wird die besagte Strömungsverbindung dadurch erreicht, daß man am Spritzenkolben einen kurzen zylindrischen Ansatz anbringt, der einen geringeren Durchmesser hat als der Kolben im Bereich der Dichtwulste, wobei dieser Ansatz auf seinem Umfang, dort wo er sich zum eigentlichen Kolben erweitert, mindestens drei voneinander etwa gleich beabstandete, auf gleicher Höhe liegende noppenartige Vorsprünge aufweist, die sich etwa soweit über den Umfang des Ansatzes erheben, wie die Dichtwulste des Kolbens.

Der Durchmesser des zylindrischen Ansatzes ist vorzugsweise etwa 10 - 25 % kleiner als der Durchmesser des Kolbens im Bereich der Dichtwulste.

Ein derartiger Spritzenkolben kann leicht mit dem besagten zylindrischen Ansatz in die kolbenseitige Öffnung einer normalen Injektionsspritze eingesetzt und durch leichtes Andrücken mit den noppenartigen Vorsprüngen im Spritzenende so fixiert werden, daß zwischen der Innenwand des Spritzenzylinders und dem Umfang des Kolbenansatzes ein deutlicher Ringspalt verbleibt, durch den beim Lyophilisieren die Dämpfe aus dem Inneren der Spritze entweichen können. Selbstverständlich darf zu diesem Zweck der Kolben nicht so weit in das Spritzenende hineingeschoben werden, daß der erste Dichtwulst sich an die Innenwand des Spritzenzylinders anlegt.

Die Verbindung zwischen Spritzeninnenraum und der Außenatmosphäre kann zusätzlich noch dadurch verbessert werden, daß man in der Umfangsfläche des Kolbenansatzes mindestens zwei axial verlaufende und am spritzenseitigen Ende des Ansatzes austretende rillenartige Vertiefungen anbringt, die kurz oberhalb der Ebene, auf der sich die noppenartigen Vorsprünge befinden, also dicht unterhalb des ersten Dichtwulstes, enden.

Anstelle der noppenartigen Vorsprünge kann man natürlich auch axial verlaufende rippenartige Vorsprünge auf dem Umfang des Kolbenansatzes anbringen, um den Kolben mit seinem Ansatzstück im kolbenseitigen Ende des Spritzenzylinders zu fixieren.

In einer anderen Ausführungsform handelt es sich bei diesen Strömungsverbindungen um in die Zylinderinnenwand eingelassene, zum Zylinderinnenraum und zum Einfüllöffnungsrand hin offene axial verlaufende Kanäle bzw. Rillen oder aber um auf die Zylinderinnenwand aufgesetzte axial verlaufende Rippen. Vorzugsweise sind diese Kanäle bzw. Rippen oder aber die Rippen ein wenig kürzer als der Kolben hoch ist. Sie sind aber nicht wesentlich kürzer als der Kolben hoch ist: gemeint ist die Höhe der Kolbendichtfläche. Es handelt sich hierbei insbesondere um eine sogenannte 2-Kammer-Spritze, die mindestens einen Beipaß zwischen den Kammern aufweist, der in dem Augenblick in Aktion tritt wo der die Kammern trennende Kolben in den Bereich des Beipasses geschoben worden ist, so daß über den Beipaß an dem Zwischenkolben vorbei ein Vermischen des Inhalts der beiden Spritzenkammern möglich ist.

Der Beipaß in der Wand des Spritzenzylinders muß nicht streng axial verlaufen. Er kann auch in einem Winkel von etwa 10 - 45° zur axialen Richtung angeordnet sein, also zur Längsrichtung der Spritze geneigt in der Zylinderwand verlaufen, so daß die Flüssigkeit aus der zweiten Kammer nicht direkt zum nadelseitigen Ende der Spritze schießt, sondern spiralig an der Spritzeninnenwand entlangläuft und sich besser mit dem Inhalt der ersten Kammer vermischt.

Die Kanäle, Rillen oder Rippen am kolbenseitigen Ende der Spritze sind so ausgebildet, daß der Spritzenkolben oder der Zwischenkolben, der im Falle einer 2-Kammer-Spritze die beiden Kammern voneinander trennt, nach dem Einfüllen des zu lyophilisierenden Gutes in die Spritze eingesetzt werden kann, so daß er im kolbenseitigen Ende der Spritze einwandfrei positioniert und gehalten ist und diese weite Öffnung weitgehend verschließt, andererseits aber über die Kanäle bzw. Rillen in diesem Endbereich der Spritzenzylinderwand an dem eingesetzten Kolben vorbei ein Entweichen der Dämpfe, die bei der Gefriertrocknung aus dem Spritzeninneren austreten, möglich ist.

Einen analogen Effekt kann man durch kurze axial verlaufende Rippenstückchen, die am kolbenseitigen Ende des Spritzenzylinders angeordnet sind, erreichen, wenn man die Höhe der Rippen und die Härte des Kolbenmaterials so aufeinander abstimmt, daß das elastische Material des Kolbens nicht völlig an den Rippenseitenwänden und an der Basis der Rippen an der Zylinderinnenwand anliegt, so daß parallel zu den Rippen Luftschlitze verbleiben. Diese Luftschlitze werden in dem Augenblick verschlossen, wo der Spritzenkolben etwas weiter in den Spritzenzylinder hinein geschoben wird, über den Bereich hinaus, der die Kanäle, Rillen oder Rippen aufweist.

Das kolbenseitige Ende des Spritzenzylinders kann aber auch so gestaltet sein, daß die besagten Rippen sich nicht über die Zylinderinnenwand erheben, sondern eine gerade Verlängerung der Zylinderinnenwand darstellen und stattdessen die Zylinderinnenwand in diesem Bereich sich um den Betrag erweitert, den die Rippenhöhe darstellt, so daß bei eingesetztem Kolben dieser von den Rippen gehalten wird und zwischen Kolbenaußenwand und Spritzenzylinderinnenwand in diesem Endbereich des Spritzenzylinders ein ringförmiger Zwischenraum entsteht, durch den bei der Gefriertrocknung die Dämpfe des Spritzeninhaltes abgezogen werden können. Auch in diesem Falle wird diese Entlüftungsmöglichkeit in dem Augenblick verschlossen, wo der Kolben über den Endbereich hinaus etwas tiefer in den Zylinder hineingeschoben wird, so daß er an der gesamten Zylinderinnenwand anliegt.

Die erfindungsgemäße Ausgestaltung einer Lyophilisierspritze, insbesondere einer 2-Kammer-Spritze, hat den Vorteil, daß man einen Spritzenzylinder mit fertig vorbereitetem Nadelansatzstück üblicher Bauart und im Falle einer 2-Kammer-Spritze auch mit einem üblichen Beipaß verwenden kann. Das Nadelansatzstück kann einen sogenannten Luerkonus aufweisen, der mit einer Kappe und/oder einem Filtereinsatz verschlossen sein kann. Füllt man in einen derartigen Spritzenkörper die zu lyophilisierende Arzneimittelkomponente ein, so kann die Spritze in der üblichen Weise mit nach unten hängendem nadelseitigem Ende in der Gefriertrocknungsanlage und außerhalb davon manipuliert werden und nach Beendigung der Gefriertrocknung durch leichtes Eindrucken des bereits in die Spritze eingesetzten Kolbens in der Gefriertrocknungsanlage dicht verschlossen werden, so daß alle nachfolgenden Handhabungen außerhalb der Gefriertrocknungsanlage vorgenommen werden können, ohne den Trockenheitsgrad des Lyophilisates durch Luftzutritt zu verändern.

Einige erfindungsgemäße Ausführungsbeispiele sollen anhand der nachfolgenden Figuren 1 bis 3 erläutert werden.
- Figur 1: zeigt einen Längsschnitt durch eine erfindungsgemäße 2-Kammer-Spritze mit einer Aufsicht auf das kolbenseitige Ende dieser Spritze;
- Figur 2: zeigt einen Teil-Längsschnitt durch ein erfindungsgemäßes Spritzenendstück mit angeformten Rippen sowie eine Aufsicht auf das kolbenseitige Ende dieser Spritze;
- Figur 3: zeigt eine weitere erfindungsgemäße Ausführungsform mit dazugehöriger Aufsicht auf das kolbenseitige Ende der Spritze.
- Figur 4: zeigt einen erfindungsgemäßen Spritzenkolben in der Seitenansicht und unmittelbar darunter das kolbenseitige Ende einer üblichen Injektionsspritze, teilweise im Axialschnitt.

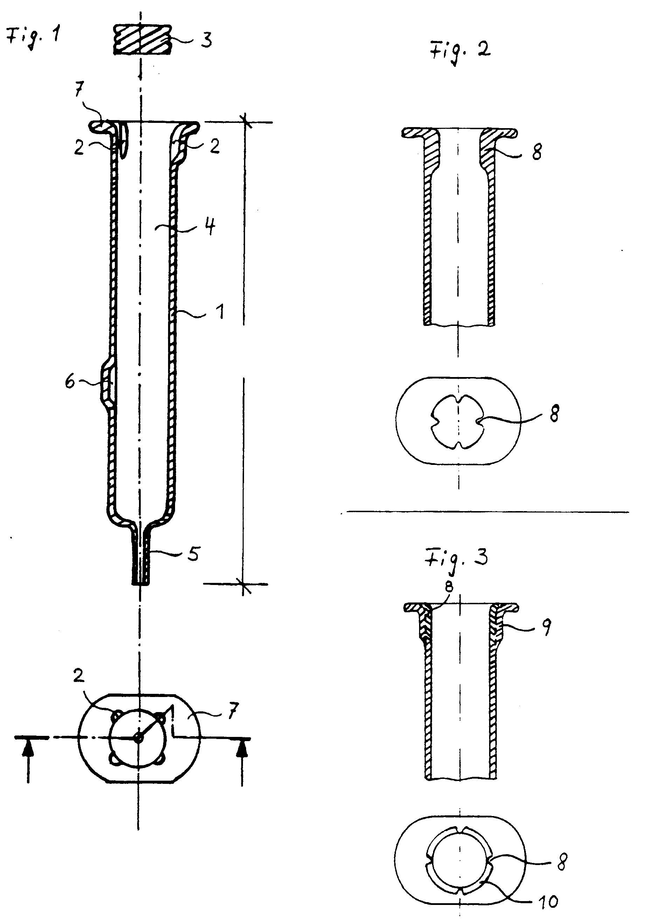

Wie in Figur 1 im Schnittbild und in der Aufsicht auf das kolbenseitige Ende einer erfindungsgemäßen LyophilisierSpritze dargestellt, sind in das kolbenseitige Ende des Spritzenzylinders 1 einige Kanäle 2 bzw. Rillen eingelassen, die sowohl zum Innenraum 4 des Spritzenzylinders 1 als auch zur Einfüllöffnung hin offen sind. Diese Kanäle bzw. Rillen verlaufen axial und erstrecken sich ein kleines Stück in Richtung nadelseitiges Ende der Spritze. Sie sind etwa so lang, wie der Spritzenkolben 3 hoch ist, vorzugsweise sind sie ein wenig kürzer als der Kolben 3 hoch ist. Wenn es sich um eine sogenannte 2-Kammer-Spritze handelt, wie in Figur 1 dargestellt, orientiert sich die Länge der Kanäle bzw. Rillen 2 an der Höhe des sogenannten Zwischenkolbens 3, das ist derjenige Kolben, der nach dem Einfüllen der ersten Komponente die erste Kammer von der zweiten Kammer etwas oberhalb des Beipasses 6 trennt. Die Spritze kann ein übliches Nadelansatzstück 5, z.B. einen Luer-Konus aufweisen, der eine übliche Verschlußkappe aufweisen kann und/oder einen Filtereinsatz oder-aufsatz haben kann. Die Spritze kann in einer üblichen Abfüllvorrichtung, bei der das spritzenseitige Ende nach unten hängt, z.B. mit einer Arzneimittellösung beschickt und anschließend mit einem üblichen Kolben 3 versehen werden. Der Kolben 3 wird in diesem Stadium nur so weit in den Spritzenzylinder eingeführt, daß die in die Spritze hineinführenden Enden der Kanäle 2 noch frei liegen, also vom Kolben nicht bedeckt sind. In diesem Zustand, das heißt mit verschlossenem Nadelansatz, eingebrachtem Lyophilisiergut und aufgesetztem Kolben 3,wird die Spritze in die Gefriertrocknungsanlage überführt. Die bei der Gefriertrocknung aus dem Lyophilisiergut abgezogenen Dämpfe entweichen über die Kanäle 2 am Kolben 3 vorbei. Sobald der Gefriertrocknungsprozeß abgeschlossen ist, wird der Kolben 3 so weit in den Spritzenzylinder hineingedrückt, daß die Kanäle 2 verschlossen sind. Wenn die Kanäle 2 etwas kürzer sind als der Kolben 3 hoch ist, wird dieser Verschluß bereits dadurch erreicht, daß der Kolben 3 durch das Zusammenfahren der Stellplatten der Gefriertrocknungsanlage mit dem Spritzenende bündig gedrückt wird. Die Spritze kann in diesem Zustand den Gefriertrockner verlassen. Im Falle einer 2-Kammer-Spritze wird man nach Entnahme aus der Gefriertrocknungsanlage den Zwischenkolben 3 weiter in das Innere der Spritze schieben, bis dicht oberhalb der Stelle, wo der Beipaß 6 endet, sodann wird die zweite Komponente, meist das Lösungsmittel für das Lyophilisat, eingefüllt, und der Injektionskolben eingeführt.

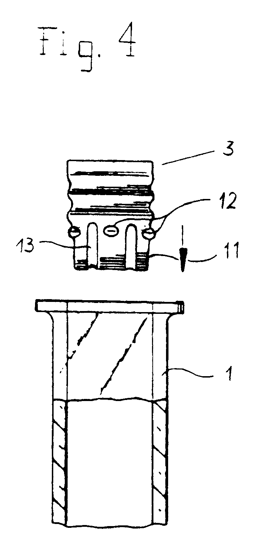

In der in Figur 2 dargestellten Ausführungsform wird die Positionierung und Fixierung des Kolbens 3 und die Entlüftung des Zylinder-Raums 4 dadurch erreicht, daß man am kolbenseitigen Ende kurze Rippen 8 anbringt.

Diese Rippen 8 können sich entweder über die Zylinderinnenwand 1 erheben, wie in Figur 2 dargestellt oder sie können mit der Zylinderinnenwand fluchten, wie in Figur 3 dargestellt. Bei der in Figur 2 dargestellten Ausführungsform wird der Kolben 3 beim Einsetzen durch die vorspringenden Rippen 8 verformt, dabei legt er sich nicht vollständig an die Rippenseitenwände an und es verbleiben beidseitig von jeder Rippe 8 parallel zur Rippe Luftschlitze, die den Innenraum 4 mit dem umgebenden Raum verbinden so lange der Kolben 3 nicht ganz in das Spritzenende eingeführt worden ist. Der Kolben 3 muß natürlich in seinem Außendurchmesser mindestens dem Innendurchmesser des Spritzenzylinders ohne Rippen entsprechen. Er kann einen kurzen Ansatz geringeren Durchmessers aufweisen, mit dem er leicht in das Spritzenende eingesetzt und zwischen den Rippen 8 positioniert und fixiert werden kann. Durch den Ringspalt zwischen der Außenwand des kurzen Ansatzes und der Innenwand des Spritzenendes können beim Gefriertrocknen die Dämpfe aus der Spritze entweichen.

Vorteilhafter ist die in Figur 3 gezeigte Ausführungsform, bei der die Rippen 8 eine Fortsetzung der Zylinderinnenwand 1 darstellen und die Zylinderinnenwand im Endbereich 9 erweitert ist, so daß bei eingesetztem Kolben 3 ein Ringraum 10 entsteht, durch den bei der Gefriertrocknung die Dämpfe aus dem Inneren der Spritze entweichen können.

Für die Abmessungen der Rippen 8 und das Verhältnis der Länge zum Kolben 3 gilt das gleiche, was vorstehend bereits im Zusammenhang mit Figur 1 gesagt wurde. Das gleiche gilt für die Handhabung dieser Ausführungsformen.

Die Zahl der Kanäle 2 oder der Rippen 8 richtet sich nach deren Durchlaßkapazität und der Menge des abzuziehenden Dampfes. Sie wird in der Regel zwischen 3 und 8, vorzugsweise bei 3 bis 5 liegen.
Als Trennkolben zwischen den beiden Kammern einer Zweikammerspritze kann vorteilhaft ein Kolben verwendet werden, der in seiner dem Lyophilisat zugewandten Dichtlippe schräge Durchlässe und zwischen dieser und der darauffolgenden Dichtlippe einen ringförmigen Zwischenraum (zwischen Zylinderinnenwand und Kolbenaußenwand) geringer Tiefe aufweist. Ein derartiger Kolben wirkt als Verteiler für das Lösungsmittel, wenn letzteres bei Betätigung der Spritze im Bereich des Beipasses in den ringförmigen Zwischenraum ein und durch die schrägen Durchlässe in das Lyophilisat austritt.

Die in Figur 4 gezeigte Ausführungsform ist besonders vorteilhaft, denn in dieser Ausführungsform kann ein üblicher, unveränderter Spritzenzylinder verwendet werden.
Der Spritzenkolben 3 ist in diesem Falle mit einem zylindrischen Ansatz 11 geringeren Durchmessers versehen. Der Durchmesser des Ansatzes ist etwa 10 - 25 % geringer als der Durchmesser des Kolbens im Bereich eines Dichtwulstes und damit natürlich auch geringer als der Innendurchmesser des Spritzenzylinders. Der Kolben 3 wird mit diesem Ansatz 11 im kolbenseitigen Ende des Spritzenzylinders mit Hilfe von noppenartigen Vorsprüngen 12 fixiert, indem man den Kolben nur so weit in das Ende des Spritzenzylinders hineindrückt, daß die noppenartigen Vorsprünge den Innenrand des Spritzenzylinders erfassen. In dieser Stellung gibt es einen Ringspalt zwischen dem Ansatz 11 und der Innenwand des Spritzenzylinders. Durch diesen Ringspalt können beim Lyophilisieren die Dämpfe aus dem Inneren der Spritze entweichen. Der mit einem solchen Ansatz versehene Kolben kann 1 - 3 Dichtwülste aufweisen. Wird er in einer Zweikammer-Spritze verwendet, muß seine dichtende Länge natürlich etwas kürzer sein als der Beipaß in der Spritzenwand. Der in Figur 4 dargestellte Kolben weist außerdem noch axial verlaufende rillenartige Vertiefungen im Umfang des Ansatzes 11 auf, um das Ent- bzw. Belüften des Zylinderinnenraumes bei aufgesetztem Kolben noch zu verbessern.
Auch in diesem Falle darf natürlich der Kolben nicht so weit in das Spritzenende eingeschoben werden, daß der erste Dichtwulst den Ringspalt bzw. die Belüftungskanäle 13 verschließt.

Die erfindungsgemäße Spritze bietet wesentliche Vorteile bei der Lyophilisierung gegenüber den bekannten Lyophilisier-Spritzen, die am nadelseitigen Ende offen sind und nach der Lyophilisierung dort durch Einfügen eines Nadelansatzstückes verschlossen werden müssen, was umständliche Operationen im Sterilbereich erfordert. Demgegenüber genügt bei der erfindungsgemäßen Spritze ein leichtes Bündigdrücken des Kolbens 3 mit dem kolbenseitigen Ende der Spritze, um nach Beendigung der Gefriertrocknung die Spritze zu verschließen.

## Patentansprüche

1. Lyophilisier-Injektionsspritze für medizinische Zwecke mit einem mindestens einen verschiebbaren Spritzenkolben (3) aufweisenden Spritzenzylinder (1), der an seinem einen Ende ein Nadelansatzstück (5) und an seinem dem Nadelansatzstück gegenüberliegenden Ende eine Öffnung zum Einführen des bzw. der Spritzenkolben(s) aufweist, **dadurch gekennzeichnet,** daß das kolbenseitige Ende des Spritzenzylinders (1) Mittel aufweist, die bei eingesetztem aber nicht völlig in den Spritzenzylinder eingeführtem Kolben (3) mindestens eine Strömungsverbindung (2, 10) zwischen Zylinderinnenraum (4) und äußerer Umgebung gewährleisten.

2. Lyophilisier-Injektionsspritze nach Anspruch 1, **dadurch gekennzeichnet,** daß es sich bei den Mitteln, welche die Strömungsverbindung (2) gewährleisten, um in die Zylinderinnenwand eingelassene, zum Zylinderinnenraum (4) und zum Einfüllöffnungsrand (7) hin offene, axial verlaufende Kanäle bzw. Rillen (2) oder aber um auf die Zylinderinnenwand aufgesetzte, axial verlaufende Rippen (8) handelt.

3. Lyophilisier-Injektionsspritze nach Anspruch 2, **dadurch gekennzeichnet,** daß die Kanäle bzw. Rillen (2) oder aber die Rippen (8) ein wenig kürzer sind als der Kolben (3) hoch ist.

4. Lyophilisier-Injektionsspritze für medizinische Zwecke mit einem mindestens einen verschiebbaren Spritzenkolben (3) aufweisenden Spritzenzylinder (1), der an seinem einen Ende ein Nadelansatzstück (5) und an seinem dem Nadelansatzstück gegenüberliegenden Ende eine Öffnung zum Einführen des bzw. der Spritzenkolben(s) aufweist,
**dadurch gekennzeichnet,**
daß der Spritzenkolben (3) einen zylindrischen Ansatz (11) aufweist, der einen geringeren Durchmesser hat als der Kolben (3) im Bereich der Dichtwulste, und daß der Ansatz (11) auf seinem Umfang, dort wo er in den Kolben übergeht, mindestens drei voneinander etwa gleich beabstandete, auf gleicher Höhe liegende noppenartige oder axial verlaufende rippenartige Vorsprünge (12) aufweist, die sich etwa soweit über den Umfang des Ansatzes (11) erheben, wie die Dichtwulste des Kolbens so daß der Kolben (3) in die Öffnung eingesetzt und durch leichtes Andrücken fixiert werden kann, um bei eingesetztem aber nicht völlig in den Spritzenzylinder eingeführtem Kolben (3) mindestens eine Strömungsverbindung (13) zwischen Zylinderinnenraum (4)und äußerer Umgebung zu gewährleisten.

5. Lyophilisier-Injektionsspritze nach Anspruch 4, **dadurch gekennzeichnet,** daß der Kolben (3) in der Umfangsfläche des Ansatzes (11) mindestens zwei axial verlaufende und am spritzenseitigen Ende des Ansatzes austretende, rillenartige Vertiefungen (13) aufweist, die kurz oberhalb der Ebene, auf der sich die noppenartigen Vorsprünge (12) befinden, enden.

6. Lyophilisier-Injektionsspritze nach einem der vorhergehenden Ansprüche, **dadurch gekennzeichnet,** daß es sich um eine sogenannte Zwei-Kammer-Spritze handelt, die mindestens einen Bypasss (6) in der Wand des Spritzenzylinders aufweist.

7. Lyophilisier-Injektionsspritze nach Anspruch 6, **dadurch gekennzeichnet,** daß der Bypass (6) nicht streng in axialer Richtung, sondern in einem Winkel von etwa 10-45° zur axialen Richtung, parallel zur Zylinderwand verläuft.

## Claims

1. Lyophilization injection syringe for medical purposes having a syringe cylinder (1) comprising at least one displaceable syringe plunger (3) and having at one end a needle attachment portion (5) and, at the end remote from the needle attachment portion, an opening for the insertion of the or each syringe plunger, characterized in that the plunger end of the syringe cylinder (1) comprises means which guarantee at least one flow connection (2,10) between the interior (4) of the cylinder and the environment when the plunger (3) is inserted but not pushed fully into the syringe cylinder.

2. Lyophilization injection syringe according to claim 1, characterized in that the means which guarantee the flow connection (2) are actually extending channels or grooves (2) let into the inner wall of the cylinder and open towards the interior (4) of the cylinder and towards the edge (7) of the fill opening or they are axially extending ribs (8) formed on the inner wall of the cylinder.

3. Lyophilization injection syringe according claim 2, characterized in that the channels or grooves (2) of the ribs (8) are slightly shorter than the height of the plunger (3).

4. Lyophilization injection syringe for medical purposes having a syringe cylinder (1) comprising at least one displaceable syringe plunger (3) and having at one end a needle attachment portion (5) and, at the end remote from the needle attachment portion, an opening for the insertion of the or each syringe plunger, characterized in that the syringe plunger (3) has a cylindrical attachment (11) which is smaller in diameter than the plunger (3) in the region of the sealing bead and in that the attachment (11) has, on its periphery, at the point where it merges into the plunger (3), at least three, substantially equally spaced knoblike or axially extending riblike projections (12) located at the same height, which project substantially the same amount above the periphery of the attachment (11) as the sealing bead of the plunger, such that the plunger (3) can be inserted into the opening and fixed by slight pressing to guarantee at least one flow connection (13) between the interior (4) of the cylinder and the environment when the plunger (3) is inserted but not pushed fully into the syringe cylinder.

5. Lyophilization injection syringe according to claim 4, characterized in that the plunger (3) has, in the circumferential surface of the attachment (11), at least two axially extending, groove-like depressions (13) emerging at the syringe and of the attachment, these depressions ending just above the plane where the knoblike projections (12) are located.

6. Lyophilization injection syringe according to one of the preceding claims, characterized in that it is a so-called two-chamber syringe which has at least one bypass (6) in the wall of syringe cylinder.

7. Lyophilization injection syringe according to claim 6, characterized in that the bypass (6) extends not strictly in the axial direction, but at an angle of about 10 to 45°C to the axial direction, parallel to the cylinder wall.

## Revendications

1. Seringue d'injection de lyophilisation destinée à des buts médicaux pourvue d'un cylindre de seringue (1) comportant au moins un piston (3) de seringue coulissant qui présente, à sa première extrémité, un élément d'appendice pour aiguille (5) et, à son extrémité opposée à l'élément d'appendice pour aiguille, une ouverture destinée à l'introduction du ou des piston(s) de seringue,
caractérisée
en ce qu'il comporte sur l'extrémité côté piston du cylindre de seringue (1), des moyens qui garantissent au moins une liaison d'écoulement (2,10) entre l'espace intérieur (4) du cylindre et son environnement extérieur lorsque le piston (3) est inséré dans le cylindre de seringue mais n'y est pas totalement introduit.

2. Seringue d'injection de lyophilisation selon la revendication 1, caractérisée en ce qu'il s'agit, pour les moyens qui garantissent la liaison (2) d'écoulement, soit de canaux ou de rainures (2) s'étendant suivant la direction axiale, ménagés dans la paroi intérieure du cylindre, ouverts vers l'espace intérieur (4) du cylindre et vers le bord (7) d'orifice de remplissage, soit de nervures (8) s'étendant suivant la direction axiale, formées sur la paroi intérieure du cylindre.

3. Seringue d'injection de lyophilisation selon la revendication 2, caractérisée en ce que, soit les canaux ou rainures (2), soit les nervures (8) sont un peu plus courts que la hauteur du piston (3).

4. Seringue d'injection de lyophilisation destinée à des buts médicaux pourvue d'un cylindre de seringue (1) comportant au moins un piston (3) de seringue coulissant qui présente, à sa première extrémité, un élément d'appendice pour aiguille (5) et, à son extrémité opposée à l'élément d'appendice pour aiguille, une ouverture destinée à l'introduction du ou des piston(s) de seringue,
caractérisée en ce que le piston (3) de seringue présente un appendice cylindrique (11), dont le diamètre est un peu plus petit que le piston (3) dans la zone des bourrelets d'étanchéité, et en ce que l'appendice (11) comporte sur sa périphérie, là ou il se transforme en piston (3), au moins trois saillies (12) en forme de boutons ou de nervures s'etendent suivant la direction axiale, situées à la même hauteur, à peu près équidistantes entre elles, qui font saillie par rapport à la périphérie de l'appendice (11) à peu près dans la même mesure que les bourrelets d'étanchéité du piston, de sorte que le piston (3) est propre à être introduit dans l'ouverture et à être fixé par une pression legère pour garantir au moins une liaison d'écoulement (13) entre le space interieur (4) du cylindre et son environnement exterieur lorsque le piston (3) est inséré dans le cylindre de seringue mais n'y est pas totalement introduit.

5. Seringue d'injection de lyophilisation selon la revendication 4, caractérisée en ce que le piston (3) comporte, dans la surface périphérique de l'appendice (11), au moins deux évidements (13) en forme de rainures, s'ètendant suivant la direction axiale, et débouchant vers l'extrémité coté seringue de l'appendice, qui se terminent un peu au-dessus du plan sur lequel se trouvent les saillies (12) en forme de boutons.

6. Seringue d'injection de lyophilisation selon l'une des revendications précédentes, caractérisée en ce qu'il s'agit de ce que l'on appelle une seringue à deux chambres, qui comporte au moins un bypass (6) dans la paroi du cylindre de seringue.

7. Seringue d'injection de lyophilisation selon la revendication 6, caractérisée en ce que le bypass (6) ne s'étend pas rigoureusement selon la direction axiale, mais selon un angle d'environ 10 à 45° par rapport à la direction axiale, parallèlement à la paroi du cylindre.
